(19) Europäisches Patentamt
European Patent Office
Office européen des brevets

(11) **EP 2 876 436 A1**

(12) **EUROPÄISCHE PATENTANMELDUNG**

(43) Veröffentlichungstag:
**27.05.2015 Patentblatt 2015/22**

(21) Anmeldenummer: **14194707.7**

(22) Anmeldetag: **25.11.2014**

(51) Int Cl.:
*G01N 29/024* (2006.01) *B29C 35/02* (2006.01)
*G01N 33/44* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **26.11.2013 DE 102013113063**

(71) Anmelder: **Deutsches Zentrum für Luft- und Raumfahrt e.V.**
**51147 Köln (DE)**

(72) Erfinder: **Liebers, Nico**
**38102 Braunschweig (DE)**

(74) Vertreter: **Aisch, Sebastian**
**Gramm, Lins & Partner GbR**
**Patent- und Rechtsanwaltssozietät**
**Freundallee 13a**
**30173 Hannover (DE)**

(54) **Verfahren zur Herstellung eines Faserverbundbauteils**

(57)     Die Erfindung betrifft ein Verfahren zur Herstellung eines Faserverbundbauteils, das aus einem mit einem Matrixmaterial infiltrierten Faserhalbzeug durch Aushärtung des Matrixmaterials hergestellt wird, wobei die Laufzeit zweier Schallsignale an unterschiedlichen Schallmessstrecken gemessen wird, wobei eine der Schallmessstrecken eine mit Matrixmaterial gefüllte Zusatzkavität aufweist, so dass sich aus den Laufzeiten der beiden Schallsignale und der Streckenlänge der Zusatzkavität die Schallgeschwindigkeit und Laminatdicke berechnen lässt.

Figur 1

EP 2 876 436 A1

**Beschreibung**

Die Erfindung betrifft ein Verfahren zur Herstellung eines Faserverbundbauteils, das aus einem mit einem Matrixmaterial infiltrierten Faserhalbzeug durch Aushärtung des Matrixmaterials hergestellt wird. Die Erfindung betrifft ebenso ein Formwerkzeug hierzu.

Bauteile aus einem Faserverbundwerkstoff, sogenannte Faserverbundbauteile, sind aus der Luft- und Raumfahrt heute nicht mehr wegzudenken. Aber auch im Automobilbereich und im Bereich der erneuerbaren Energie (z.B. Windkraftanlagen) findet die Verwendung derartiger Werkstoffe immer mehr Zuspruch. Insbesondere kritische Strukturelemente werden aufgrund der hohen gewichtsspezifischen Festigkeit und Steifigkeit bei minimalem Gewicht aus faserverstärkten Kunststoffen gefertigt. Durch die aus der Faserorientierung resultierenden anisotropen Eigenschaften der Faserverbundwerkstoffe können Bauteile exakt an lokale Belastung angepasst werden und ermöglichen so eine optimale Materialausnutzung im Sinne des Leichtbaus.

Beim herkömmlichen Injektionsverfahren wird bei der Herstellung eines Faserverbundbauteils in trockenes Fasermaterial mit einem Matrixmaterial infiltriert, indem das Matrixmaterial in ein Fasermaterial, das sich in der Regel in einem Formwerkzeug zwecks Formgebung befindet, injiziert wird. Üblicherweise wird das Formwerkzeug zusammen mit dem Faserhalbzeug in einen Autoklaven eingebracht, damit unter Beaufschlagung von Druck und Temperatur das Matrixmaterial in das Fasermaterial des Faserhalbzeuges injiziert werden kann und anschließend das so mit dem Matrixmaterial infiltrierte Faserhalbzeug ausgehärtet werden kann.

Dabei ist es wünschenswert, den Injektions- und Aushärtungsprozess zu überwachen, um so die vorgegebenen Randbedingungen des herzustellenden Faserverbundbauteils prozesssicher einhalten zu können. Dabei stellt sich die Schwierigkeit, dass aufgrund des in sich abgeschlossenen Autoklavprozesses eine Überwachung der maßgeblichen Messgrößen nur bedingt möglich ist.

Es ist bekannt, mit Hilfe von Ultraschall den Aushärtungsprozess von Faserverbundbauteilen während der Aushärtung zu überwachen. Dabei wird von einem Ultraschallwandler bzw. einem Ultraschallgeber ein Ultraschallimpuls durch das Faserhalbzeug gesendet und von einem gegenüberliegenden Empfänger bzw. Sensor detektiert. Anhand der gemessenen Zeit, die der Ultraschallimpuls durch das Faserhalbzeug benötigt, lässt sich die Schallgeschwindigkeit des Matrixmaterials ableiten, die mit zunehmender Vernetzung des Matrixmaterials und somit zunehmendem Fortschritt des Aushärtungsprozesses steigt. Daher lässt sich anhand der Schallgeschwindigkeit des Matrixmaterials sehr gut der Fortschritt des Aushärtungsprozesses feststellen.

Ein Beispiel hierfür ist die DE 197 37 276 A1, die ein Verfahren und eine Vorrichtung zur Ultraschall-Überwachung der physikalischen und chemischen Eigenschaften von Duroplasten bei der Verarbeitung offenbart. Mit Hilfe eines Referenz-Schallsignals, das in das Bauteil gesendet wird, lässt sich zusammen mit dem empfangenen Schallsignal unter Anwendung von Korrelationstechniken Eigenschaften der verarbeiteten Werkstoffe ableiten.

Die Anwendung der Ultraschalltechnik bei der Herstellung von Faserverbundbauteilen zur Ermittlung der Schallgeschwindigkeit ist sinnvoll doch nur dann möglich, wenn zwei starre Formhälften ("closed-mould") eines Formwerkzeuges verwendet werden, da dann eine definierte und konstante Bauteildicke vorgegeben ist, die als Grundlage zur Berechnung der Schallgeschwindigkeit aus den Laufzeiten der Ultraschallimpulse dient. Bei sogenannten "open-mould"-Verfahren, bei denen nur eine starre Formseite vorhanden ist, während die zweite Formseite in der Regel flexibel und durch eine Vakuumfolie abgedeckt ist, ist die konkrete Bauteildicke des Faserverbundbauteils während des Aushärtungsprozesses nicht genau bekannt, da sie großen Schwankungen während des Infusions- und Aushärtungsprozesses unterliegt. Damit ist im Ultraschallmesssystem nicht nachvollziehbar, ob die Laufzeitänderung durch die Vernetzung des Harzes oder einer Dickenänderung hervorgerufen wird. Daher kann auch keine genaue Schallgeschwindigkeit berechnet werden, wodurch keine Aussage über den aktuellen Vernetzungsgrad getroffen werden kann.

Bei diesen "open-mould"-Verfahren ist die Bauteildicke, die in der Regel die Messrichtung des Ultraschallimpulses darstellt, aufgrund der Beschaffenheit des Fasermaterials, der Menge des in das Fasermaterial infiltrierten Matrixmaterials und durch den Druck im Autoklaven veränderlich. Mangels Messmethoden zur Bestimmung der Bauteildicke im Prozess kann die Bauteildicke jedoch nur nach Durchlaufen des Infusions- und Aushärtungsprozesses am fertigen Bauteil gemessen werden. Die optimalen Prozessparameter zum Erreichen einer gezielten Bauteildicke muss also in einer aufwendigen Versuchsreihe gefunden werden. Die zahlreichen Einflussparameter auf dem Infusionsprozess und die Bauteildickenentwicklung können dennoch nicht erfasst werden, wodurch auch bei optimierten Prozessparametern eine hohe Bauteildickentoleranz auftritt.

Es ist Aufgabe der vorliegenden Erfindung ein verbessertes Verfahren und eine verbesserte Vorrichtung zur Herstellung eines Faserverbundbauteils anzugeben, mit dem insbesondere im "open-mould"-Verfahren die physikalischen und chemischen Eigenschaften des Faserverbundbauteils während des Infusions- und Aushärtungsprozesses sicher und exakt überwacht werden können.

Die Aufgabe wird mit dem Verfahren gemäß Anspruch 1 sowie mit der Fertigungsvorrichtung gemäß Anspruch 7 erfindungsgemäß gelöst.

Gemäß Anspruch 1 wird ein Verfahren zur Herstellung eines Faserverbundbauteils vorgeschlagen, bei dem zunächst, wie aus dem Stand der Technik bekannt, die Laufzeit eines ersten Schallsignals einer ersten Schallmessstrecke mittels eines ersten Schallmesssystems ermittelt wird. Hierfür wird das erste Schallsignal in das mit dem Matrixmaterial infiltrierten Faserhalbzeug emittiert. Es ist nicht zwangsläufig notwendig, dass das Faserhalbzeug bereits vollständig mit dem Matrixmaterial infiltriert wurde. Es sollte jedoch der Bereich des Faserhalbzeuges der von der ersten Schallmessstrecke betroffen ist, mit dem Matrixmaterial infiltriert sein.

Erfindungsgemäß wird nun vorgeschlagen, dass die Laufzeit eines zweiten Schallsignals einer zweiten Schallmessstrecke mittels eines zweiten Schallmesssystems ermittelt wird. Im Unterschied zu der ersten Schallmessstrecke wird dabei das zweite Schallsignal in das mit dem Matrixmaterial infiltrierten Faserhalbzeug und in eine mit dem Matrixmaterial gefüllte Zusatzkavität, die Bestandteil der zweiten Schallmessstrecke ist, emittiert, so dass das zweite Schallsignal wenigstens durch das mit dem Matrixmaterial infiltrierten Faserhalbzeug und die zusätzlich vorgesehene und mit dem Matrixmaterial gefüllte Zusatzkavität gesendet wird. Mit anderen Worten, das zweite Schallsignal durchläuft sowohl das mit dem Matrixmaterial infiltrierte Faserhalbzeug als auch die mit dem Matrixmaterial gefüllte Zusatzkavität.

Erfindungswesentlich ist hierbei, dass die mit dem Matrixmaterial gefüllte Zusatzkavität nicht Bestandteil der ersten Schallmessstrecke ist, so dass die Laufzeit des ersten Schallsignals sich aus der Schallmessstrecke ohne die Zusatzkavität ergibt.

Aufgrund der Tatsache, dass die Abmessungen der verwendeten Zusatzkavität bekannt sind, lässt sich unter Kenntnis der Laufzeit des ersten Schallsignals, der Laufzeit des zweiten Schallsignals und der Streckenlänge des die Zusatzkavität betreffenden Teils der zweiten Schallmessstrecke die Schallgeschwindigkeit des Matrixmaterials durch eine Auswerteeinheit während des Infusions- und/oder Aushärtungsprozesses berechnen.

So lässt sich beispielsweise die Schallgeschwindigkeit des Matrixmaterials mittels der folgenden Formel berechnen

$$c = \frac{\Delta d_2}{t_2 - t_1}$$

$$(1)$$

, wobei c die Schallgeschwindigkeit ist, $\Delta d_2$ die Streckenlänge des die Zusatzkavität betreffenden Teils der zweiten Schallmessstrecke ist, $t_1$ die Laufzeit des ersten Schallsignals und $t_2$ die Laufzeit des zweiten Schallsignals darstellen. Vorteilhafterweise handelt es sich bei den Laufzeiten der Schallsignale um die sogenannten Netto-Laufzeiten, d.h. abzüglich eventueller Sensorvorläufe. Die Netto-Laufzeit des ersten Schallsignals betrifft somit nur die Laufzeit des Schallsignals innerhalb des mit dem Matrixmaterial infiltrierten Faserhalbzeuges, während die Netto-Laufzeit des zweiten Schallsignals die Laufzeit des Schallsignals in dem mit Matrixmaterial infiltrierten Faserhalbzeug und die mit Matrixmaterial gefüllte Zusatzkavität betrifft. Die Differenz der Laufzeiten kann alternativ auch aus den beiden gemessenen Signalen bestimmt werden.

Durch die Anordnung zweier paralleler Schallmessstrecken, wobei eine der beiden Schallmessstrecken eine definierte Zusatzkavität mit Matrixmaterial enthält, kann auf die Laufzeit eines Schallsignals innerhalb der Zusatzkavität bezüglich der vorgegebenen Streckenlänge der Zusatzkavität geschlossen werden, woraufhin sich die Schallgeschwindigkeit des Matrixmaterials nahezu exakt berechnen lässt. Hierdurch lässt sich auch innerhalb der "open-mould"-Verfahren bei der Herstellung von Faserverbundbauteilen anhand der Schallgeschwindigkeit der Fortschritt der Vernetzung des Matrixmaterials während des Aushärtungsprozesses überwachen, so dass der gesamte Infusions- und Aushärtungsprozess online überwachbar wird.

Vorteilhafterweise liegen die beiden Schallmessstrecken relativ dicht beieinander, um so lokale Unterschiede in der Streckenlänge innerhalb des Faserhalbzeuges zu minimieren. Es ist außerdem besonders vorteilhaft, wenn die Steckenlänge des die Zusatzkavität betreffenden Teils der zweiten Schallmessstrecke kleiner ist als die Streckenlänge des das Faserhalbzeug betreffenden Teils der zweiten Schallmessstrecke.

Unter einem Faserhalbzeug im Sinne der vorliegenden Erfindung wird das noch nicht ausgehärtete Faserverbundbauteil verstanden, das zumindest Fasermaterial aufweist. Unter dem Faserhalbzeug wird auch das mit dem Matrixmaterial infiltrierte Fasermaterial in Kombination verstanden. Denkbar hierbei wären auch bereits vorimprägnierte Faserhalbzeuge.

Ein Schallsignal kann beispielsweise ein Ultraschallimpuls sein, der mit Hilfe eines Ultraschallgebers in das Faserhalbzeug emittiert, d.h. ausgesendet wird. Dabei können grundsätzlich die beiden bekannten Schallverfahren eingesetzt werden: Durchschallungsverfahren oder Impuls-Echo-Verfahren. Beim Durchschallungsverfahren wird auf der einen Seite ein Schallgeber angeordnet, der ein Schallsignal in das Faserhalbzeug emittiert, während auf der gegenüberliegenden Seite ein Schallsensor vorgesehen ist, der das emittierte Schallsignal nach dem Durchlaufen des Schallsignals durch das Faserhalbzeug empfängt. Beim Impuls-Echo-Verfahren ist der Schallgeber und Schallsensor identisch, wobei sich hier insbesondere piezoelektrische Schallelemente eignen.

Die Zusatzkavität weist insbesondere in formstabiles Material auf, das sich unter Einwirkung von Druck und Temperatur beispielsweise innerhalb eines Autoklaven, nicht oder nur sehr wenig verformt, unabhängig von der physikalischen Wärmeausdehnung. Hierbei ist es zweckmäßig, dass die Zusatzkavität aus dem gleichen Material wie das Formwerkzeug besteht.

Vorteilhafterweise lässt sich mit Hilfe der Auswerteeinheit auch eine Streckenlänge des das Faserhalbzeug betreffenden Teils der ersten Schallmessstrecke in Abhängigkeit von der Laufzeit des ersten Schallsignals und der berechneten Schallgeschwindigkeit des Matrixmaterials berechnen, so dass sich aufgrund der erfindungsgemäß berechneten Schallgeschwindigkeit des Matrixmaterials die Bauteildicke, die parallel zu den Schallmessstrecken ist, berechnen lässt. Dies kann beispielsweise mit Hilfe der folgenden Formel erfolgen

$$d_1 = c \cdot t_1 = \frac{\Delta d \cdot t_1}{|t_2 - t_1|}$$

$$(2)$$

wobei $d_1$ die Streckenlänge des das Faserhalbzeug betreffenden Teils der ersten Schallmessstrecke ist, c die Schallgeschwindigkeit und $t_1$ die Laufzeit des ersten Schallsignals darstellen.

Es lässt sich auch die Streckenlänge des das Faserhalbzeug betreffenden Teils der zweiten Schallmessstrecke berechnen, wobei hier die Streckenlänge durch die Zusatzkavität herausgerechnet werden muss. Somit stellt dies ein Äquivalent zu den Berechnungen der Streckenlänge des das Faserhalbzeug betreffenden Teils der ersten Schallmessstrecke dar.

Unter einer Schallmessstrecke im Sinne der vorliegenden Erfindung wird insbesondere die Haupt-Ausbreitungsrichtung bzw. der Haupt-Ausbreitungsweg zwischen dem Sender und dem Empfänger verstanden. Beim Durchschallungsverfahren bedeutet dies, dass die Schallmessstrecke insbesondere der Weg durch das Faserhalbzeug ist, der sich als gedachte Linie zwischen dem Sender und dem Empfänger ergibt. Beim Impuls-Echo-Verfahren ist die Schallmessstrecke der Weg vom Schallgeber insbesondere durch das Faserhalbzeug bis zu dem reflektierenden Bereich und dann wieder zurück zum Schallgeber, der dann als Sensor fungiert. Die Schallmessstrecke ist somit jener Weg des Schallimpulses, der zwischen Sender und Empfänger liegt. In der Regel ist dies die kürzeste Verbindung.

Anhand der berechneten Schallgeschwindigkeit und alternativ oder zusätzlich auch anhand der Streckenlänge des das Faserhalbzeug betreffenden Teils der ersten Schallmessstrecke kann dann während des Herstellungsprozesses die Prozessparameter des Herstellungsprozesses durch eine Steuereinheit eingestellt werden. Hierdurch wird es beispielsweise möglich, die Faserhalbzeugdicke auch bei schwankenden Randbedingungen (Flächengewicht des Fasermaterials, Fließeigenschaften des Harzes, Fließfrontverlauf) durch Regelung der Prozessparameter gezielt und reproduzierbar einzustellen. So kann die Faserhalbzeugdicke beispielsweise unter anderem durch Druckänderung im Autoklaven oder durch gezieltes Öffnen und Schließen der Matrixmaterialleitungen gesteuert werden. Darüber hinaus können durch die berechnete Schallgeschwindigkeit Rückschlüsse auf den Aushärtungsgrad des Matrixmaterials geschlossen werden, so dass die eigentliche Autoklav-Zykluszeit exakt an die vorherrschenden Bedingungen angepasst werden kann.

Vorteilhafterweise wird die Zusatzkavität an oder in dem Faserhalbzeug in der zweiten Schallmessstrecke derart angeordnet, dass während der Injektion des Matrixmaterials in das Fasermaterial des Faserhalbzeuges die Zusatzkavität ebenfalls mit dem injizierten Matrixmaterial gefüllt wird. Hierdurch kann sichergestellt werden, dass während des Injektionsvorgangs das in das Fasermaterial des Faserhalbzeuges injizierte Matrixmaterial auch die Zusatzkavität füllt, so dass das vorliegende Verfahren sichere Ergebnisse liefert. Hierfür kann beispielsweise ein Kavitätswerkzeug vorgesehen sein, das die Zusatzkavität enthält und mit der offenen Seite die Zusatzkavität auf das Faserhalbzeug gelegt wird. Während der Injektion des Matrixmaterials kann so das Matrixmaterial aus dem Faserhalbzeug in die Zusatzkavität einfließen und diese somit befüllen.

In weiteren vorteilhaften Ausführungsformen wird für die erste Schallmessstrecke ein erster Sensorvorlauf und für die zweite Schallmessstrecke ein zweiter Sensorvorlauf ermittelt, um so die Netto-Laufzeiten der Schallsignale zu ermitteln. Hierfür wird anhand eines Referenz-Schallsignals die Laufzeit des Schallsignals außerhalb des das Faserhalbzeug betreffenden Teils der ersten Schallmessstrecke berechnet, so dass die Laufzeit des ersten Schallsignals abzüglich des ersten Sensorvorlaufes ermittelbar wird. Die Laufzeit des ersten Schallsignals betrifft dann somit lediglich die Laufzeit des Schallsignals in dem Faserhalbzeug selbst.

Genauso wird auch die Laufzeit des zweiten Schallsignals ermittelt, wobei hierfür ein zweiter Sensorvorlauf der zweiten Schallmessstrecke ermittelt wird, wobei hier jedoch in Abhängigkeit von einem Referenz-Schallsignal die Laufzeit des Schallsignals sowohl außerhalb des das Faserhalbzeug betreffenden Teils der zweiten Schallmessstrecke und außerhalb des die Zusatzkavität betreffenden Teils der zweiten Schallmessstrecke berechnet wird. Die Laufzeit der zweiten Schallmessstrecke wird dann abzüglich dieses zweiten Sensorvorlaufes ermittelt, wobei somit die Laufzeit des zweiten Schallsignals die reine Laufzeit des Schallsignals in dem Faserhalbzeug und in der Zusatzkavität enthält.

Hierdurch wird es möglich, dass die Schallmesssysteme außen an ein Formwerkzeug angebracht werden können, so dass die Schallgeber und Schallempfänger nicht zwangsläufig direkt mit dem Faserhalbzeug in Kontakt treten müssen. Der Sensorvorlauf betrifft dabei insbesondere die Strecke durch das Formwerkzeug sowie durch das Kavitätswerkzeug der Zusatzkavität, auf der beispielsweise ein Teil des Schallmesssystems angeordnet sein kann.

Die Erfindung wird anhand der beigefügten Figuren beispielhaft erläutert. Es zeigen:

Figur 1     - Schematische Darstellung einer Fertigungsvorrichtung im Funktionsprinzip des Durchschallungs-Verfahrens;

Figur 2     - Schematische Darstellung einer Fertigungsvorrichtung im Funktionsprinzip des Impuls-Echo-Verfahrens;

Figur 3     - Schematische Darstellung einer Ausführungsform mit ringförmigem Sensor.

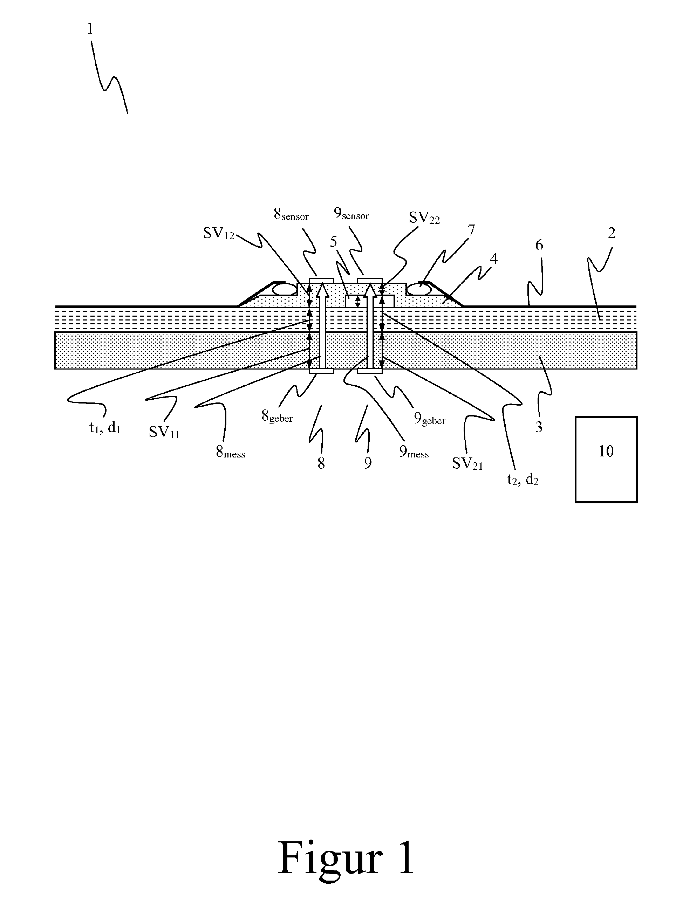

Figur 1 zeigt eine Fertigungsvorrichtung 1, mit der ein Faserverbundbauteil aus einem Faserhalbzeug 2 hergestellt werden soll. Im vorliegenden Beispiel ist das Faserhalbzeug 2 aus mehreren Lagen Fasermaterial in ein Formwerkzeug 3 eingebracht. Im Injektionsprozess, wobei die dafür benötigten Bestandteile nicht dargestellt sind, wird das Matrixmaterial in das Faserhalbzeug 2 injiziert, um so das Fasermaterial des Faserhalbzeuges 2 vollständig mit Matrixmaterial zu benetzen.

Gegenüberliegend des Formwerkzeuges 3 wird auf das Faserhalbzeug 2 ein Kavitätswerkzeug 4 angeordnet, das die für das erfindungsgemäße Verfahren benötigte Zusatzkavität 5 enthält. Die Zusatzkavität 5 des Kavitätswerkzeuges 4 ist dabei in Richtung des Faserhalbzeuges 2 an einer Seite offen und steht somit mit dem Faserhalbzeug 2 kommunizierend in Verbindung, so dass während des Injektionsprozesses das in das Faserhalbzeug 2 injizierte Matrixmaterial auch in die Zusatzkavität 5 des Kavitätswerkzeuges 4 injiziert wird. Für den vollständigen Vakuumaufbau ist des Weiteren eine Vakuumfolie 6 vorgesehen, die das Faserhalbzeug 2 gegenüber dem Formwerkzeug 3 vakuumdicht verschließen soll. Im Bereich der Vakuumfolie 6 ist dabei das Kavitätswerkzeug 4 vorgesehen, wobei mittels einer Dichtung 7 die Vakuumfolie 6 und das Kavitätswerkzeug 4 das Faserhalbzeug 2 vakuumdicht gegenüber dem Formwerkzeug 3 abdichtet.

Mittels eines derartigen Aufbaus lassen sich nun die herkömmlichen Infusionsund Aushärtungsprozesse zur Herstellung eines Faserverbundbauteils durchführen, bei denen zunächst das Faserhalbzeug 2 evakuiert, dann das Matrixmaterial injiziert und anschließend das injizierte Matrixmaterial in dem Faserverbundbauteil ausgehärtet wird. Bei der Injektion des Matrixmaterials in das Faserhalbzeug 2 wird dabei auch die Zusatzkavität 5 des Kavitätswerkzeuges 4 mit dem Matrixmaterial gefüllt.

Erfindungsgemäß sind nun ein erstes Schallmesssystem 8 und ein zweites Schallmesssystem 9 vorgesehen, mit denen Schallsignale, beispielsweise Ultraschallimpulse, in das mit Matrixmaterial gefüllte Faserhalbzeug 2 und die mit Matrixmaterial gefüllte Zusatzkavität 5 emittiert werden können. Im Ausführungsbeispiel der Figur 1 weist jedes der beiden Schallmesssysteme 8 und 9 jeweils einen Schallgeber und einen Schallsensor auf, so dass das Funktionsprinzip der Durchschallung Anwendung findet. Hierfür sind die Schallgeber an der Unterseite des Formwerkzeuges 3 angeordnet, während die Schallsensoren an der Oberseite des Kavitätswerkzeuges 4 angeordnet sind.

Das erste Schallmesssystem 8 weist somit an der Unterseite des Formwerkzeuges 3 einen Schallgeber $8_{Geber}$ auf, der ein Schallsignal in Richtung des an dem Kavitätswerkzeug 4 angeordneten Schallsensors $8_{Sensor}$ emittiert. Hierdurch wird eine erste Schallmessstrecke $8_{Mess}$ bereitgestellt, bei der ein Schallsignal durch das Formwerkzeug 3, durch das Faserhalbzeug 2 und durch das Kavitätswerkzeug 4 durchschalt wird, bis es von dem Schallsensor $8_{Sensor}$ empfangen wird.

Unter Berücksichtigung der Sensorvorläufe $SV_{11}$ und $SV_{12}$ lässt sich dann von dem ersten Schallsignal die Laufzeit $t_1$ berechnen. Die Berechnung der Sensorvorläufe $SV_{11}$ und $SV_{12}$ kann beispielsweise dadurch geschehen, dass mit Hilfe von einem Referenz-Schallsignal die Laufzeit ermittelt wird, und zwar dann, wenn kein Faserhalbzeug 2 in das Formwerkzeug 3 eingelegt wurde und das Kavitätswerkzeug 4 direkt auf dem Formwerkzeug 3 gekoppelt mit Hilfe eines Koppelmaterials wie beispielsweise Wasser aufliegt. Anhand der Laufzeit des Referenz-Schallsignals lässt sich dann der Sensorvorlauf $SV_{11}$ und $SV_{12}$ ermitteln.

Die Laufzeit $t_1$ des ersten Schallsignals der ersten Schallmessstrecke $8_{Mess}$ lässt sich dann wie folgt berechnen:

$$t_1 = t_{gesamt} - SV_{11} - SV_{12}$$

(3)

wobei $t_{gesamt}$ die Gesamtlaufzeit zwischen dem Schallgeber und dem Schallsensor ist, $SV_{11}$ der Sensorvorlauf im Formwerkzeug 3 und $SV_{12}$ der Sensorvorlauf im Kavitätswerkzeug 4 darstellt.

In unmittelbarer Nähe zu der ersten Schallmessstrecke 8 $_{Mess}$ wird eine zweite Schallmessstrecke 9 $_{Mess}$ mit Hilfe des zweiten Schallmesssystems 9 errichtet, wobei auch hier ein Schallgeber 9 Geber an dem Formwerkzeug 3 angeordnet ist, während der Schallsensor 9 Sensor an dem Kavitätswerkzeug 4 angeordnet ist.

Die zweite Schallmessstrecke 9 $_{Mess}$ ist dabei so eingerichtet, dass das Schallsignal zumindest in das Faserhalbzeug 2 und in die Zusatzkavität 5 des Kavitätswerkzeuges 4 emittiert wird. Im vorliegenden Beispiel der Figur 1 wird somit das Schallsignal durch das Formwerkzeug 3, dann durch das Faserhalbzeug 2, dann durch die Zusatzkavität 5 und dann durch den Rest des Kavitätswerkzeuges 4 gesendet bzw. durchgeschallt. Auch hier können die Sensorvorläufe $SV_{21}$ und $SV_{22}$ betreffend das Formwerkzeug 3 und das Kavitätswerkzeug 4 im Vorfeld mit Hilfe eines Referenz-Schallsignals ermittelt werden, um so die Netto-Laufzeiten des Schallsignals durch das Faserhalbzeug 2 und die Zusatzkavität 5 zu ermitteln. Für das zweite Schallsignal ergibt sich somit eine Laufzeit $t_2$ aus der Formel:

$$t_2 = t_{gesamt} - SV_{21} - SV_{22}$$

(4)

wobei $t_{gesamt}$ die Gesamtlaufzeit des Schallsignals vom Geber zum Sensor ist, $SV_{21}$ der Sensorvorlauf des Formwerk-zeuges 3 und $SV_{22}$ der Sensorvorlauf des Kavitätswerkzeuges ist.

Aufgrund der Tatsache, dass die Abmessungen der Zusatzkavität im Vorfeld bekannt sind und keinen Schwankungen unterliegen, kann die Schallgeschwindigkeit gemäß

$$c = \frac{\Delta d_2}{\left| t_2 - t_1 \right|}$$

(1)

berechnet werden, wobei $\Delta d_2$ die bekannte Streckenlänge der Zusatzkavität 5 darstellt, welche den Teil der zweiten Schallmessstrecke 9 $_{Mess}$ betrifft.

Hieraus lässt sich dann im Bereich der ersten Schallmessstrecke 8 $_{Mess}$ die Streckenlänge $d_1$ des Teils des Faserhalbzeuges 2 ermitteln, der die erste Schallmessstrecke 8 $_{Mess}$ betrifft.

$$d_1 = c \cdot t_1 = \frac{\Delta d \cdot t_1}{\left| t_2 - t_1 \right|}$$

(2)

Im vorliegenden Fall betrifft die Streckenlänge $d_1$ die Dicke des Faserhalbzeuges 2 das aus mehreren Lagen Fasermaterial aufgebaut ist. Mittels des vorliegenden Verfahrens lässt sich somit während des Infusions- und Aushärtungsprozesses exakt die Streckenlänge $d_1$ im vorliegenden Fall also die Laminatdicke, berechnen und durch Anpassen der Prozessparameter während der Herstellung entsprechend korrigieren, wenn die Laminatdicke vom Soll abweicht. Mit Hilfe der permanent ermittelten Schallgeschwindigkeit kann darüber hinaus der Fortschritt des Aushärtungsprozesses sicher überwacht werden.

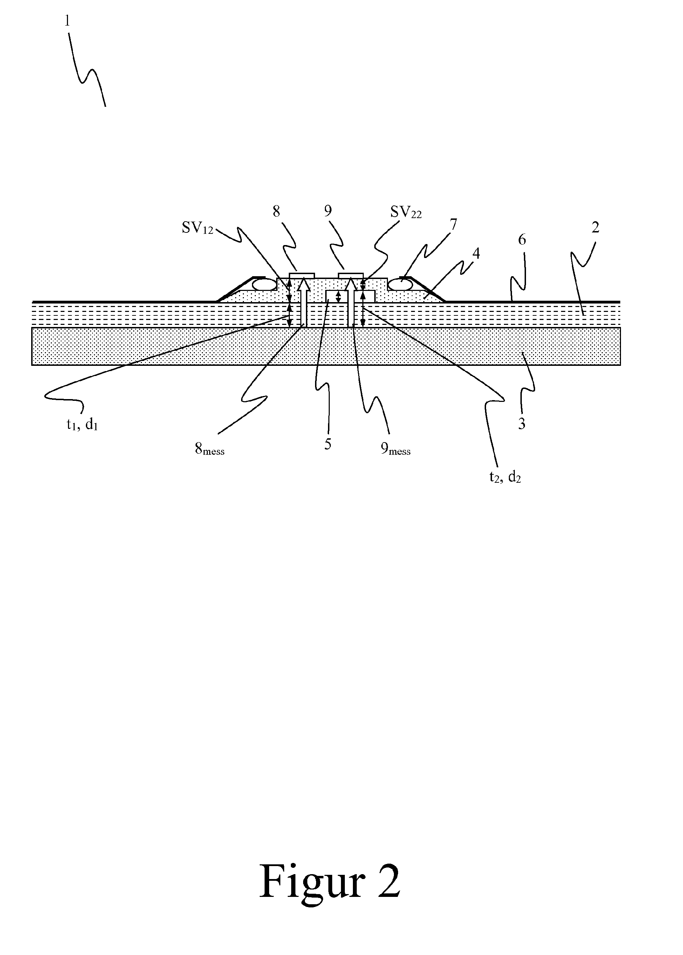

Figur 2 zeigt schematisch eine Fertigungsvorrichtung, bei der das Funktionsprinzip des Echo-Impuls-Verfahrens Anwendung findet. Hierbei wird nur ein Sensor pro Schallmesssystem verwendet, der gleichzeitig auch als Empfänger dient. Im Ausführungsbeispiel der Figur 2 ist dieser Sensor/Empfänger an dem Kavitätswerkzeug 4 angeordnet und wird dabei an der Grenzschicht zwischen Faserhalbzeug 2 und Formwerkzeug 3 reflektiert, so dass das Echo durch den Sensor wieder empfangbar wird. Ein Sensorvorlauf bezüglich des Formwerkzeuges 3 braucht dann nicht berücksichtigt werden.

Die Zusatzkavität 5 sollte so beschaffen sein, dass sie während der Infusion mit Harz gefüllt wird und durch Formschrägen eine Entformung nach der Aushärtung möglich ist.

Mit der vorliegenden Erfindung wird es möglich, auch bei den sogenannten "o-pen-mould"-Verfahren eine Aushärtungskontrolle des Matrixmaterials durchzuführen und gleichzeitig die Laminatdicke zu bestimmen. Hierfür bedarf es keines fixen Schätzwertes der Laminatdicke für die Berechnung der Schallgeschwindigkeit, wobei die Zusatzkavität auch in Form eines Stempels ausgeführt sein kann. Damit lassen sich die Absolutwerte von Schallgeschwindigkeit und Laminatdicke während des gesamten Infusions- und Aushärtungsprozesses ermitteln.

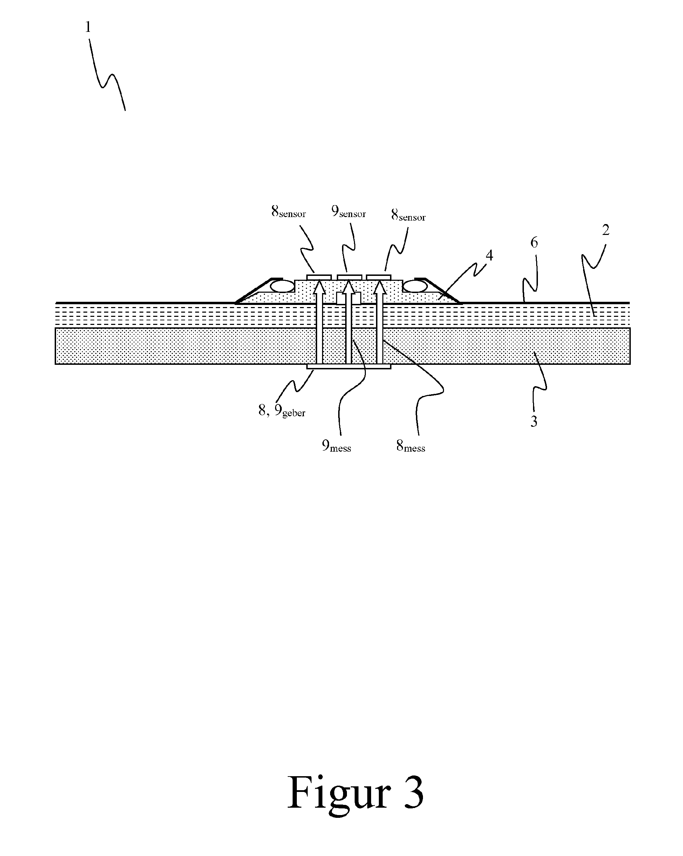

Figur 3 zeigt schematisch eine Ausführungsform, bei der das zweite Schallmesssystem von dem ersten Schallmesssystem umfasst wird. Hierbei weisen das erste Schallmesssystem und das zweite Schallmesssystem ein und denselben Schallgeber 8, 9 Geber auf, der kreisförmig ausgebildet ist. Der am anderen Ende der jeweiligen Schallmessstrecke angeordnete Sensor ist dabei bei dem ersten Schallmesssystem ringförmig ausgebildet, so dass in deren mittleren Öffnung der Schallsensor 9 Sensor des zweiten Schallmesssystems angeordnet werden kann. Das zweite Schallmesssystem ist somit von dem ersten Schallmesssystem umfasst.

Diese Ausführungsform hat den Vorteil, dass für die beiden Schallmesssysteme nur ein einziger Schallgeber vorgesehen ist, wobei die Schallsensoren aufgrund der ringförmigen Ausbildung des ersten Schallmesssystems dicht beieinander angeordnet werden können, so dass die erste Schallmessstrecke und die zweite Schallmessstrecke dicht beieinander liegen.

**Bezugszeichenliste**

| | |
|---|---|
| 1 | - Fertigungsvorrichtung |
| 2 | - Faserhalbzeug |
| 3 | - Formwerkzeug |
| 4 | - Kavitätswerkzeug |
| 5 | - Zusatzkavität |
| 6 | - Vakuumfolie |
| 7 | - Dichtung |
| 8 | - erstes Schallmesssystem |
| $8_{Geber}$ | - Schallgeber des ersten Schallmesssystems |
| $8_{Sensor}$ | - Sensor des ersten Schallmesssystems |
| $8_{Mess}$ | - erste Schallmessstrecke |
| 9 | - zweites Schallmesssystem |
| $9_{Geber}$ | - Schallgeber des zweiten Schallmesssystems |
| $9_{Sensor}$ | - Schallsensor des zweiten Schallmesssystems |

| $9_{Mess}$ | - zweite Schallmessstrecke |
|---|---|
| $t_1$ | - Laufzeit des ersten Schallsignals |
| $d_1$ | - Streckenlänge des das Faserhalbzeug betreffenden Teils der ersten Schallmessstrecke |
| $SV_{11}$, $SV_{12}$ | - Sensorvorlauf der ersten Schallmessstrecke |
| $t_2$ | - Laufzeit des zweiten Schallsignals |
| $d_2$ | - Streckenlänge des das Faserhalbzeug und die Zusatzkavität betreffenden Teils der zweiten Schall- messstrecke |
| $SV_{21}$, $SV_{22}$ | - Sensorvorlauf der zweiten Schallmessstrecke |
| $\Delta d_2$ | - bekannte Streckenlänge des die Zusatzkavität betreffenden Teils der zweiten Schallmessstrecke. |
| Ai/au | |

**Patentansprüche**

1. Verfahren zur Herstellung eines Faserverbundbauteils, das aus einem mit einem Matrixmaterial infiltrierten Faserhalbzeug durch Aushärtung des Matrixmaterials hergestellt wird, mit den Schritten:

   a) Ermitteln der Laufzeit ($t_1$) eines ersten Schallsignals einer ersten Schallmessstrecke ($8_{mess}$) mittels eines ersten Schallmesssystems (8), wobei das ersten Schallsignal in das mit dem Matrixmaterial infiltrierten Faserhalbzeug emittiert wird,
   **gekennzeichnet durch**
   b) Ermitteln der Laufzeit ($t_2$) eines zweiten Schallsignals einer zweiten Schallmessstrecke ($9_{mess}$) mittels eines zweiten Schallmesssystems (9), wobei das zweite Schallsignal in das mit dem Matrixmaterial infiltrierten Faserhalbzeug und in eine mit dem Matrixmaterial gefüllte Zusatzkavität (5), die Bestandteil der zweiten Schallmessstrecke ist, emittiert wird, wobei die mit dem Matrixmaterial gefüllte Zusatzkavität nicht Bestandteil der ersten Schallmessstrecke ist, und
   c) Berechnen der Schallgeschwindigkeit (c) des Matrixmaterials in Abhängigkeit von der Laufzeit ($t_1$) des ersten Schallsignals, der Laufzeit ($t_2$) des zweiten Schallsignals und der Streckenlänge ($\Delta d_2$) des die Zusatzkavität betreffenden Teils der zweiten Schallmessstrecke **durch** eine Auswerteeinheit.

2. Verfahren nach Anspruch 1, **dadurch gekennzeichnet, dass** eine Streckenlänge ($d_1$) des das Faserhalbzeug betreffenden Teils der ersten Schallmessstrecke in Abhängigkeit von der Laufzeit des ersten Schallsignals und der berechneten Schallgeschwindigkeit des Matrixmaterials durch die Auswerteeinheit berechnet wird.

3. Verfahren nach Anspruch 1 oder 2, **dadurch gekennzeichnet, dass** während des Herstellungsprozesses die Prozessparameter des Herstellungsprozesses in Abhängigkeit von der während des Herstellungsprozesses kontinuierlich berechneten Schallgeschwindigkeit und/oder Streckenlänge des das Faserhalbzeug betreffenden Teils der ersten Schallmessstrecke durch eine Steuereinheit eingestellt werden.

4. Verfahren nach einem der vorhergehenden Ansprüche, **dadurch gekennzeichnet, dass** die Zusatzkavität an oder in dem Faserhalbzeug in der zweiten Schallmessstrecke derart angeordnet wird, das während der Injektion des Matrixmaterials in das Fasermaterial des Faserhalbzeuges die Zusatzkavität mit dem injizierten Matrixmaterial gefüllt wird.

5. Verfahren nach einem der vorhergehenden Ansprüche, **dadurch gekennzeichnet, dass** ein erster Sensorvorlauf ($SV_{11}$, $SV_{12}$) der ersten Schallmessstrecke ermittelt wird, indem in Abhängigkeit von einem Referenz-Schallsignal die Laufzeit eines Schallsignals außerhalb des das Faserhalbzeug betreffenden Teils der ersten Schallmessstrecke berechnet wird, wobei die Laufzeit des ersten Schallsignals abzüglich des ersten Sensorvorlaufes ermittelt wird.

6. Verfahren nach einem der vorhergehenden Ansprüche, **dadurch gekennzeichnet, dass** ein zweiter Sensorvorlauf ($SV_{21}$, $SV_{22}$) der zweiten Schallmessstrecke ermittelt wird, indem in Abhängigkeit von einem Referenz-Schallsignal die Laufzeit eines Schallsignals außerhalb des das Faserhalbzeug betreffenden Teils und des die Zusatzkavität betreffenden Teils der zweiten Schallmessstrecke berechnet wird, wobei die Laufzeit des zweiten Schallsignals abzüglich des zweiten Sensorvorlaufes ermittelt wird.

7. Fertigungsvorrichtung (1) zur Herstellung eines Faserverbundbauteils, das aus einem mit einem Matrixmaterial infiltrierten Faserhalbzeug durch Aushärtung des Matrixmaterials hergestellt wird, mit

- einem Formwerkzeug (3), das eine formgebende Oberfläche zum Ablegen des Faserhalbzeuges hat,
- einem Kavitätswerkzeug (4), das eine Zusatzkavität (5) zum Befüllen mit Matrixmaterial aufweist, wenn das Kavitätswerkzeug an dem in das Formwerkzeug eingebrachten Faserhalbzeug angeordnet ist,
- einem ersten Schallmesssystem (8), das eine erste Schallmessstrecke zum Emittieren eines ersten Schallsignals in das mit Matrixmaterial infiltrierten Faserhalbzeug bildet und zum Ermitteln der Laufzeit des ersten Schallsignals ausgebildet ist,
- einem zweiten Schallmesssystem (9), das eine zweite Schallmessstrecke zum Emittieren eines zweiten Schallsignals in das mit Matrixmaterial infiltrierten Faserhalbzeug und in die mit Matrixmaterial gefüllte Zusatzkavität bildet und zum Ermitteln der Laufzeit des zweiten Schallsignals ausgebildet ist, wobei die mit Matrixmaterial gefüllte Zusatzkavität nicht Bestandteil der ersten Schallmessstrecke ist, und
- einer Auswerteeinheit (10), die zum Berechnen der Schallgeschwindigkeit des Matrixmaterials in Abhängigkeit von der Laufzeit des ersten Schallsignals, der Laufzeit des zweiten Schallsignals und der Streckenlänge des die Zusatzkavität betreffenden Teils der zweiten Schallmessstrecke eingerichtet ist.

8. Fertigungsvorrichtung nach Anspruch 7, **dadurch gekennzeichnet, dass** die Auswerteeinheit zum Berechnen einer Streckenlänge des das Faserhalbzeug betreffenden Teils der ersten Schallmessstrecke in Abhängigkeit von der Laufzeit des ersten Schallsignals und der berechneten Schallgeschwindigkeit des Matrixmaterials eingerichtet ist.

9. Fertigungsvorrichtung nach Anspruch 7 oder 8, **dadurch gekennzeichnet, dass** die Auswerteeinheit zum Ermitteln der Laufzeit des ersten Schallsignals abzüglich eines die erste Schallmessstrecke betreffenden ersten Sensorvorlaufes und/oder zum Ermitteln der Laufzeit des zweiten Schallsignals abzüglich eines die zweite Schallmessstrecke betreffenden zweiten Sensorvorlaufes eingerichtet ist.

10. Fertigungsvorrichtung nach einem der Ansprüche 7 bis 9, **dadurch gekennzeichnet, dass** das Kavitätswerkzeug derart ausgebildet ist, dass die Zusatzkavität mit dem in das Fasermaterial des Faserhalbzeuges injizierte Matrixmaterial gefüllt wird, wenn das Matrixmaterial in das Fasermaterial des Faserhalbzeuges injiziert wird.

11. Fertigungsvorrichtung nach einem der Ansprüche 7 bis 10, **dadurch gekennzeichnet, dass** die Fertigungsanlage eine Vakuumfolie zum vakuumdichten Verschließen des in das Formwerkzeug eingebrachten Faserhalbzeuges aufweist, wobei das Kavitätswerkzeug in einem Bereich der Vakuumfolie vorgesehen und gegenüber der Vakuumfolie vakuumdicht abgedichtet ist.

12. Fertigungsvorrichtung nach einem der Ansprüche 7 bis 11, **dadurch gekennzeichnet, dass** eine Steuereinheit vorgesehen ist, die zum Einstellen der Prozessparameter während des Herstellungsprozesses in Abhängigkeit von der kontinuierlich berechneten Schallgeschwindigkeit und/oder Streckenlänge des das Faserhalbzeug betreffenden Teils der ersten Schallmessstrecke eingerichtet ist.

Figur 1

# Figur 2

1

8_sensor    9_sensor    8_sensor

2

6

4

8, 9_geber

9_mess    8_mess

3

# Figur 3

**EUROPÄISCHER RECHERCHENBERICHT**

Nummer der Anmeldung

EP 14 19 4707

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| T | Nico Liebers: "DGZfP-Jahrestagung 2014 - In-Situ Laminatdickenmessung während der Infusion und Aushärtung von Faserverbundkunststoffen", The e-Journal of Nondestructive testing, 1. März 2015 (2015-03-01), Seiten 1-11, XP055177492, Gefunden im Internet: URL:http://www.ndt.net/search/link.php?id=17358&file=article/dgzfp2014/papers/mo3a3.pdf [gefunden am 2015-03-18] * das ganze Dokument * ----- | | INV. G01N29/024 B29C35/02 G01N33/44 |
| A,D | DE 197 37 276 A1 (DOERING JOACHIM DR [DE]; STARK WOLFGANG DR [DE]; BARTUSCH JUERGEN [DE]) 11. März 1999 (1999-03-11) * Zusammenfassung * * Abbildung 1 * ----- | 1,7 | |
| A | SCHMACHTENBERG E ET AL: "Application of ultrasonics for the process control of Resin Transfer Moulding (RTM)", POLYMER TESTING, ELSEVIER, AMSTERDAM, NL, Bd. 24, Nr. 3, 1. Mai 2005 (2005-05-01), Seiten 330-338, XP027767365, ISSN: 0142-9418 [gefunden am 2005-05-01] * Zusammenfassung * * Seite 333 * ----- -/-- | 1,7 | RECHERCHIERTE SACHGEBIETE (IPC) G01N B29C |

Der vorliegende Recherchenbericht wurde für alle Patentansprüche erstellt

| Recherchenort | Abschlußdatum der Recherche | Prüfer |
|---|---|---|
| München | 30. März 2015 | Rouault, Patrick |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**Europäisches Patentamt**
**European Patent Office**
**Office européen des brevets**

# EUROPÄISCHER RECHERCHENBERICHT

Nummer der Anmeldung

EP 14 19 4707

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | STOVEN T ET AL: "Continuous monitoring of three-dimensional resin flow through a fibre preform", COMPOSITES PART A: APPLIED SCIENCE AND MANUFACTURING, ELSEVIER SCIENCE PUBLISHERS B.V., AMSTERDAM, NL, Bd. 34, Nr. 6, 1. Juni 2003 (2003-06-01), Seiten 475-480, XP004428273, ISSN: 1359-835X, DOI: 10.1016/S1359-835X(03)00059-9 * Zusammenfassung * * Seite 476 - Seite 477 * * Abbildung 1 * ----- | 1,7 | |
| A | US 2008/315462 A1 (BATZINGER THOMAS JAMES [US] ET AL) 25. Dezember 2008 (2008-12-25) * Zusammenfassung * * Absatz [0022] * * Abbildung 1 * ----- | 1,7 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 30. März 2015 | Rouault, Patrick |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

.................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 14 19 4707

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-03-2015

| Im Recherchenbericht angeführtes Patentdokument | Datum der Veröffentlichung | Mitglied(er) der Patentfamilie | Datum der Veröffentlichung |
|---|---|---|---|
| DE 19737276 A1 | 11-03-1999 | KEINE | |
| US 2008315462 A1 | 25-12-2008 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19737276 A1